# EUROPEAN PATENT SPECIFICATION

(11) **EP 0 831 921 B1**
(45) Date of publication and mention of the grant of the patent: **02.01.2004**
(21) Application number: 96917038.0
(22) Date of filing: 03.06.1996
(51) Int. Cl.: A61K 48/00, C12N 15/86, C12N 15/49, A61K 39/12, A61K 39/118

(54) **RECOMBINANT RACCOON POX VIRUSES AND THEIR USE AS AN EFFECTIVE VACCINE AGAINST FELINE IMMUNODEFICIENCY VIRUS INFECTION**
REKOMBINANTE POCKENVIREN DES WASCHBAERS SOWIE IHRE VERWENDUNG ALS EIN EFFEKTIVER IMPFSTOFF GEGEN EINE INFEKTION MIT IMMUNSCHWAECHEVIREN DER KATZE
POXVIRUS RECOMBINES DU RATON LAVEUR ET LEUR UTILISATION EN TANT QUE VACCIN EFFICACE CONTRE L'INFECTION PAR LE VIRUS DE L'IMMUNODEFICIENCE FELINE

(30) Priority: 07.06.1995 US 482090
(43) Date of publication of application: 01.04.1998
(60) Divisional application: 03013934.9
(73) Proprietor: Wyeth, Madison, New Jersey 07940-0874 (US)
(72) Inventor: WASMOEN, Terri, Fort Dodge, IA 50501 (US); CHU, Hsien-Jue, Fort Dodge, IA 50501 (US); CHAVEZ, Lloyd, George, Jr., Highlands Ranch, CO 80126 (US)
(74) Representative: Talbott, Dawn Jacqueline
(86) International application number: PCT/US1996/008508
(87) International publication number: WO 1996/040268

(56) References cited:
- EP-A- 0 652 287
- WO-A-92/15684
- WO-A-94/02613
- WO-A-94/06471
- US-A- 5 266 313
- REVIEWS IN MEDICAL MICROBIOLOGY, vol. 4, 1993, pages 80-88, XP000600230 BAXBY, D.: "Recombinant poxvirus vaccines"
- JANIS KUBY: 'Immunology, Second Edition', W.H. FREEMAN AND CO, NEW YORK

## Description

### FIELD OF THE INVENTION

The present invention pertains to the prophylaxis of disease caused by feline immunodeficiency virus (FIV), using as vaccines recombinant raccoon poxviruses (RRPVs) expressing the envelope protein of FIV.

### BACKGROUND OF THE INVENTION

Feline immunodeficiency virus (FIV) infection is a significant health problem for domestic cats around the world. As in its human counterpart, infection with FIV causes a progressive disruption in immune function. In the acute phase of infection, the virus causes transient illness associated with symptoms such as lymphadenopathy, pyrexia, and neutropenia. Subsequently, an infected animal enters an asymptomatic phase of 1-2 years before clinical manifestations of immune deficiency become apparent, after which the mean survival time is usually less than one year.

FIV is a typical retrovirus that contains a single-stranded polyadenylated RNA genome, internal structural proteins derived from the *gag* gene product, and a lipid envelope containing membrane proteins derived from the *env* gene product (Bendinelli et al., *Clin.Microbiol.Rev*. **8**:87, 1995). The *gag* gene is translated into a primary product.of about 50 kDa that is subsequently cleaved by a viral protease into the matrix, capsid, and nucleocapsid proteins. The *env* gene yields a primary translation product of 75-80 kDa (unglycosylated molecular weight); in infected cells, the precursor has an apparent molecular weight of 145-150 kDa due to N-linked glycosylation. The *env* precursor is cleaved in the Golgi apparatus into the SU and TM proteins (also designated gp95 and gp40, respectively).

Most vaccines against FIV have failed to induce protective immunity. Ineffective vaccines have involved inactivated whole virus, fixed infected cells, recombinant CA and SU proteins, and a synthetic peptide corresponding to the V3 region of SU. In some cases, the vaccine actually enhanced infection after challenge. In one system, vaccination with paraformaldehyde-fixed virus or infected cells resulted in protective immunity (Yamamoto et al., *J. Virol.* **67**:601, 1993), but application of this approach by others was unsuccessful (Hosie et al., in *Abstracts of the International Symposium on Feline Retrovirus* Research, 1993, page 50).

WO94/06471 and WO94/02613 disclose anti-feline immunodeficiency virus (FIV) vaccines. WO92/15684 discloses recombinant FIV glycoprotein 160 and p24 GAG protein. EP 0652287 discloses poxviral vectors and their use as a vaccine against feline infectious peritonitis virus disease. US 5266313 discloses raccoon poxvirus as a gene expression and vaccine vector for genes of rabies virus and other organisms.

Thus, there is a need in the art for an effective vaccine against FIV that utilizes *env* proteins, or fragments therefrom, as immunogens.

### SUMMARY OF THE INVENTION

The present invention pertains to the prevention or lessening of disease in cats caused by Feline Immunodeficiency Virus (FIV). Prevention or lessening of disease is understood to mean the amelioration of any symptoms, including immune system disruptions that result from FIV infection.

The invention provides recombinant raccoon poxviruses having at least one internal gene comprising a DNA sequence that encodes, FIV envelope protein (*env*), a polypeptide consisting of amino acids 1-735 of FIV *env*, or immunogenic fragments of any of the foregoing. By immunogenic fragment is meant any portion of the coding sequence of FIV *gag* or *env* polypeptides that induces a beneficial immune response in cats.

A first aspect of the invention therefore relates to a recombinant raccoon poxvirus having at least one internal gene comprising a DNA sequence encoding a polypeptide consisting of amino acids 1-735 of the envelope protein of a Feline Immunodeficiency Virus (FIV).

In a preferred embodiment the internal gene of the recombinant raccoon poxvirus encodes the FIV envelope protein having the amino acid sequence as set out in SEQ. ID No. 12. In another preferred embodiment the internal gene encodes amino acids 1-735 of the FIV envelope protein having the amino acid sequence as set out in SEQ ID No. 12.

In another aspect, the invention encompasses vaccines that comprise one or more of the FIV-expressing recombinant raccoon poxviruses described above, with a pharmaceutically acceptable carrier or diluent and a pharmaceutically acceptable adjuvant.

Further aspects of the invention relate to a DNA sequence encoding amino acids 1-735 of an FIV envelope protein according to SEQ ID No. 12 and to its use in medicine.

In yet another aspect, methods for preventing or lessening disease caused by FIV, which is carried out by administering to a feline in need of such treatment the vaccines described above, are disclosed. Hence, the invention provides for the use of the recombinant raccoon poxviruses described above in the preparation of a vaccine for the prevention or lessening of disease caused by FIV.

### BRIEF DESCRIPTION OF THE DRAWINGS

Figure 1 is a graphic illustration of the cloning strategy for the envelope gene of FIV.
Figure 2 is a diagrammatic representation of the structure of the recombinant FIV env gene in pSL-EnvABC.
Figure 3 shows the DNA [SEQ. I.D. NO. 14] and protein sequence [SEQ. I.D. NO. 12] of the *env* gene of FIV.
Figure 4 is a graphic illustration of the pSL-WGag plasmid.
Figure 5 shows the DNA [SEQ. I.D. NO. 13] and protein sequence [SEQ. I.D. NO. 11] of the *gag* gene of FIV.
Figure 6 is a graphic illustration of the cloning strategy for construction of the raccoon poxvirus transfer plasmid pSC11-FIV gag.
Figure 7 is a graphic illustration of the cloning strategy for construction of the raccoon poxvirus transfer plasmid pSC11-FIV Env.
Figure 8 is a graphic illustration of the cloning strategy for construction of the raccoon poxvirus transfer plasmid pSC11-FIV EnvAB.

### DETAILED DESCRIPTION OF THE INVENTION

The vaccine of the present invention may be prepared by creating recombinant raccoon poxviruses (RRPVs) containing a gene encoding the *gag* or *env* proteins of Feline Immunodeficiency Virus (FIV) or immunogenic fragments thereof. *Gag* and *env* genes useful in practicing the present invention may be obtained by methods well-known in the art. In one embodiment, viral RNA is reverse-transcribed using endogenous or exogenous reverse transcriptase and the DNA is rendered double-stranded using DNA polymerase. The *gag* and *env*-encoding DNA segments are then recovered by restriction enzyme digestion and are amplified by cloning in *E. coli*. In another embodiment, FIV-infected cat cells serve as a source of FIV proviral DNA. In this embodiment, chromosomal DNA is isolated from the cells, and oligonucleotide primers are used to specifically amplify the *gag* and *env* genes or fragments therefrom using polymerase chain reaction techniques. This approach is broadly applicable to purifying *gag* and *env* genes from different FIV strains or isolates, since primers can be designed from non-polymorphic regions of the FIV genome.

FIV *gag* and *env* genes isolated by the above methods are first inserted into a transfer plasmid, and the recombinant plasmid is introduced into appropriate host cells that have been previously infected with a raccoon poxvirus. As a result, the DNA from the transfer plasmid is incorporated into the poxvirus DNA by homologous recombination, producing the RRPVs that are released from the cells.

DNA encoding the FIV *gag* or *env* proteins or fragments therefrom are inserted into a transfer plasmid downstream of a poxvirus promoter. In a preferred embodiment, the early/late 7.5 kD protein promoter of vaccinia virus is used. However, alternate promoter elements could be used.

The preferred transfer plasmid also contains a beta-galactosidase marker gene, which allows for selection and detection of the plasmid DNA sequences in recombinant viruses. It will be understood by those of ordinary skill in the art that alternate selectable marker genes, such as the neomycin resistance gene or the *E. coli gpt* gene or others, could be used to practice the invention. Flanking the inserted FIV gene and the selectable marker gene are thymidine kinase DNA sequences, which facilitate integration of the plasmid DNA sequences into the raccoon poxvirus DNA by homologous recombination.

Recombinant viruses expressing the FIV *gag* or *env* genes are prepared by first infecting a susceptible cell line (such as Vero [ATCC CCL 81], BSC-1 [ATCC CCL 26], RAT-2 [ATCC CRL 1764], or CRFK [ATCC CCL 94]) with wild type raccoon poxvirus (ATCC VR-838 or similar isolates). Transfer plasmid DNA containing the FIV *gag* or *env* gene is then transfected into the infected cells using cationic liposome-mediated transfection, or other suitable techniques such as electroporation or calcium-phosphate precipitation. Raccoon poxviruses incorporate DNA from the transfer plasmid through homologous recombination with the thymidine kinase gene sequences present on the plasmid. Virus infection is allowed to proceed until cytopathic effects are noted in all cells.

Incorporation of the FIV *gag* or *env* gene into poxvirus DNA is accompanied by disruption of the viral thymidine kinase gene. Thus, recombinant virus may be selected for by the absence of a thymidine kinase gene; this is achieved by selective expansion on RAT-2 cells (tk-, ATCC CRL 1764) in the presence of 5-bromodeoxyuridine. Viruses containing a gene insert from the transfer plasmid are identified by blue plaque color when grown in the presence of a chromogenic substrate for beta-galactosidase such as X-gal.

Viral plaques that survive these selection and screening procedures are then subjected to several cycles of plaque purification. Subsequently, the presence of the *gag* or *env* genes is confirmed by polymerase chain reaction technology, and the presence of *gag* or *env* antigenic determinant is confirmed by immunoblot analysis using specific antibodies. These viruses are designated by RRPV-FIV *gag* and RRPV-FIV *env,* respectively.

RRPVs can be produced that express less-than-full-length segments of the FIV *gag* and *env* proteins. The techniques used to engineer transfer plasmids encoding partial sequences of *env and gag* are well-known and widely used in the art, as are the methods for production and screening of RRPVs as detailed in this specification. For example, convenient restriction enzyme recognition sites can be used to obtain fragments of either gene, as described, e.g., Example 1 below. Alternatively, introduction of oligonucleotides containing a stop codon at various points along *gag* or *env* DNA will produce a nested set of carboxyterminal-truncated versions of that gene, which can then be incorporated into RRPVs. Furthermore, sequences that encode different domains on each protein may be recombined, using domains derived from different FIV strains or isolates. It will be apparent to one of ordinary skill in the art that systematic screening of such recombinant RRPVs can establish whether the intact protein, subfragments thereof or multi-strain recombinants thereof, are most preferred in practicing the present invention. Furthermore, as stated above, DNA encoding different fragments of *gag* and *env* can be used in a combination vaccine after incorporation into the same, or different, RRPVs.

For vaccine preparation, susceptible cells are grown in minimum essential media containing fetal bovine serum or a suitable media substitute. Cells are infected with recombinant raccoon poxvirus at a multiplicity of infection of 0.1 infectious units/cell or less. In this specification an infectious unit is defined as a Tissue Culture Infectious Dose (TCID₅₀), an amount of virus yielding 50% infection under defined conditions. When cytopathology is noted in > 90% of the cells, the infected cells and extracellular fluids are harvested. The virus may be stored frozen (-50°C or colder) or lyophilized until the time of use. Compounds such as NZ-amine, dextrose, gelatin or others designed to stabilize the virus during freezing and lyophilization may be added. The virus may be concentrated using commercially available equipment.

Typically, the concentration of virus in the vaccine formulation will be a minimum of 10^{6.5} TCID₅₀ per dose , but will typically be in the range of 10^{7.0} to 10^{9.0} TCID₅₀ per dose. At the time of vaccination, the virus is thawed (if frozen) or reconstituted (if lyophilized) with a physiologically-acceptable carrier such as deionized water, saline, phosphate buffered saline, or the like.

In one embodiment, a physiologically acceptable adjuvant such as, for example, EMA31, Adjuvant A, or combinations thereof, is added to the vaccine formulation. Non-limiting examples of suitable adjuvants include squalane and squalene (or other oils of animal origin); block copolymers such as Pluronic® (L121) Saponin; detergents such as Tween®-80; Quil® A, mineral oils such as Drakeol® or Marcol®, vegetable oils such as peanut oil; Corynebacterium-derived adjuvants such as corynebacterium parvum; Propionibacterium-derived adjuvants such as Propionibacterium acne; Mycobacterium bovis (Bacillus Calmette and Guerinn, or BCG); interleukins such as interleukin 2 and interleukin-12; monokines such as interleukin 1; tumor necrosis factor; interferons such as gamma interferon; combinations such as saponin-aluminum hydroxide or Quil®-A aluminum hydroxide; liposomes; iscom adjuvant; mycobacterial cell wall extract; synthetic glycopeptides such as muramyl dipeptides or other derivatives; Avridine; Lipid A; dextran sulfate; DEAE-Dextran or DEAE-Dextran with aluminum phosphate; carboxypolymethylene, such as Carbopol®; EMA; acrylic copolymer emulsions such as Neocryl® A640 (e.g. U.S. Patent 5,047,238); vaccinia or animal poxvirus proteins; subviral particle adjuvants such as orbivirus; cholera toxin; dimethytdiocledecylammonium bromide; or mixtures thereof.

EMA 31 (Monsanto, St. Louis, MO) is a linear ethylene/maleic copolymer with approximately equal amounts of ethylene and maleic anhydride, having an estimated average molecular weight of about 75,000 to 100,000. Adjuvant A is an adjuvant comprising a block copolymer, such as a polyoxypropylene-polyoxyethylene (POP-POE) block copolymer, preferably Pluronic® L121 (e.g. U.S. Patent 4,772,466), and an organic component, such as a metabolizable oil, e.g. an unsaturated tuipin hydrocarbon, preferably squalane (2,6,10,15,19,23-hexamethyltetracosane) or squalene. The vaccine may also include a non-ionic detergent or surfactant, preferably a polyoxyethylene sorbitan monooleate such as a Tween® detergent, most preferably Tween®-80, i.e. polyoxyethylene (20) sorbitan monooleate.

In this adjuvant mixture, the block copolymer, organic oil, and surfactant may be present in amounts ranging from about 10 to about 40 ml/L, about 20 to about 80 ml/L, and about 1.5 to about 6.5 ml/L, respectively. In a preferred embodiment of the stock adjuvant, the organic component is squalane present in an amount of about 40 mL/L, the surfactant is polyoxyethylenesorbitan monooleate (Tween®-80) present in an amount of about 3.2 ml/L, and the POP-POE block copolymer is Pluronic® L121 present in an amount of about 20 ml/L. Pluronic® L121 is a liquid copolymer at 15-40°C, where the polyoxypropylene (POP) component has a molecular weight of 3250 to 4000 and the polyoxyethylene (POE) component comprises about 10-20%, preferably 10%, of the total molecule.

Individual raccoon poxviruses expressing the *gag* or *env* genes may be mixed together for vaccination. Furthermore, the virus may be mixed with additional inactivated or attenuated viruses, bacteria, or fungi, or with immunogens derived from viruses, bacteria, or fungi such as feline leukemia virus, feline panteukopenia virus, feline rhinotracheitis virus, feline calicivirus, feline infectious peritonitis virus, feline *Chlamydia psittaci, Microsporum canis*, or others. In addition, antigens from the above-cited organisms may be incorporated into combination vaccines. These antigens may be purified from natural sources or from recombinant expression systems, or may comprise individual subunits of the antigen or synthetic peptides derived therefrom.

In a further embodiment of the present invention, live or inactivated RRPV virus-cell lysates can be incorporated into liposomes, or encapsulated in peptide-, protein-, or polysaccharide-based microcapsules prior to administration, using means that are known in the art. The vaccine of the present invention is administered to cats in volumes ranging from 0.5 to 5 milliliters. The vaccine can be administered to cats by subcutaneous, intramuscular, oral, intradermal, or intranasal routes. The number of injections and their temporal spacing may be varied. One to three vaccinations administered at intervals of one to three weeks are usually effective.

The efficacy of the vaccines of the present invention is assessed by the following methods. At about one month after the final vaccination, vaccinates and controls are each challenged with 3-20 cat ID₅₀ units, preferably 5 cat ID₅₀ units of FIV, preferably the NCSU1 isolate (ATCC VR-2333). Whole blood is obtained from the animals immediately before challenge, and at intervals after challenge, for measurement of a) viremia and b) relative amounts of CD4 and CD8 lymphocytes.

Viremia is measured by isolating mononuclear cells from the blood, and co-culturing the cells with mononuclear cells from uninfected animals. After 7 days of culture, the culture supernatants are tested for FIV by enzyme-linked immunoassay (See Example 5 below).

The ratio of CD4 to CD8 lymphocytes in the circulation of vaccinates and controls is taken as a measure of immune function. Typically, FIV infection causes an inversion of the normal CD4:CD8 ratio of about 1.5-4.0 to a pathological ratio of about 0.5-1.0. The numbers of CD4 and CD8 lymphocytes are measured by flow cytometry using specific antibodies (see Example 5 below).

Another measure of immune function is to challenge vaccinates and controls with *Toxoplasma gondii* at 6-12 months after the final RRPV-FIV vaccination. Normally, the severity of *T. gondii-* induced disease symptoms is considerably exacerbated in FIV-infected cats relative to uninfected cats. The severity of the *T. gondii* effect is determined by scoring ocular discharge, nasal discharge, dyspnea, and fever.

It will be understood that amelioration of any of the symptoms of FIV infection is a desirable clinical goal. This includes a lessening of the dosage of medication used to treat FIV-induced symptoms.

The following examples are intended to illustrate the present invention without limitation thereof.

### Example 1: Cloning of FIV gag and env Cenes

### A. Isolation of Viral DNA

FIV strain NCSU-1 (designated "FIV-NCSU-1") was isolated from a naturally infected, feline leukemia virus-negative cat and has been described previously (Tompkins et al., *J. Am. Vet. Med. Assoc*. 199: 1311, 1991. The virus was passed in a normal specific pathogen-free (SPF) cat (obtained from Liberty Laboratories, Waverly, NY). FIV-infected peripheral blood mononuclear cells (PBMC) were obtained from whole blood by separation on discontinuous percoll gradients. Briefly, anti-coagulated whole blood was layered over a two step gradient containing 43 % Percoll™ (Pharmacia, Piscataway, NJ) over 62.5% Percoll™ in 0.15 M NaCl. Gradients were centrifuged at 400 x g for 5 minutes, followed by 800 x g for 20 minutes at 22°C. PBMC were harvested from the gradient interface and washed in phosphate buffered saline containing 5 % fetal bovine serum. In parallel, PBMCs were isolated from normal cats.

FIV was propagated by co-culture of PBMCs from an FIV-infected cat with PBMCs from normal cats. The cells were maintained in RPMI 1640 media containing 10% fetal bovine serum, 2.5 x 10⁻⁵ beta-mercaptoethanol, 2 mM L-glutamine, 5 µg/mL concanavalin A, and 20% conditioned media from MLA cells (ATCC TIB 201) as a source of interleukin-2 (IL-2).

Cat genomic DNA containing FIV-NCSU-1 proviral sequences was isolated from the cultured PBMCs by lysis of the cells with 0.6% sodium dodecyl sulfate (SDS) in 10 mM Tris-HCl, pH 7.5, 10 mM EDTA, followed by precipitation of chromosomal DNA by incubation overnight with 1 mM NaCl. The DNA was recovered by centrifugation at 10,000 r.p.m. (Beckman J2, JA-20 rotor) for 40 minutes. The DNA pellet was resuspended in a solution containing 10 mM Tris-HCl pH 7.5, 10 mM EDTA, 0.1% SDS buffer and digested with ribonuclease A (20 µg/ml) and proteinase K (0.2 mg/ml) at 50°C for 4 hours. DNA was then purified by sequential extraction with phenol, phenol:chloroform (1:1) and chloroform, and was recovered in pure form followed by ethanol precipitation.

### B. Cloaing of FIV Envelope Gene

FIV-NCSU-1 envelope DNA sequences were cloned using polymerase chain reaction (PCR) methods as follows:

### Envelope Fragment A

The following oligonucleotides were used to amplify the 5' proximal segment of the *env* gene.

5'-TCGGATCCAACAATAATTATGGCAGAAGG-3' [SEQ. I.D. NO. 1] (Coding
strand, 6252-V)

5'-AATCAGGTACAAAGTCACCGTTC-3' [SEQ. I.D. NO. 2] (Complementary strand,
6745-C)

Primer 6252-V corresponds to nucleotides 6252-6273 of FIV strain PPR (GenBank No. M36968) and primer 6745-C (underlined region) corresponds to nucleotides 6723-6745 of FIV strain 14 (GenBank No. 25381). The start codon for envelope protein translation is included in primer 6252-V. Primer 6252-V also has a synthetic BamHI restriction enzyme site near the 5' end to facilitate cloning. An AvrII site located at position 6719 also facilitates cloning. Envelope fragment A is 494 bp in length.

### Envelope Fragment B

The following oligonucleotides were used to amplify the middle segment of the *env* gene.

5'-TATAGAAGCACCCCAAGAAGAG-3' [SEQ. I.D. NO. 3] (Coding strand, 6637-V)

5'-CATTCCCCCAAAGTTATATTTC-3' [SEQ. I.D. NO. 4] (Complementary strand,
8469-C)

Primers 6637-V and 8469-C correspond to nucleotides 6637-6659 and 8448-8469 of FIV 14 strain, respectively. An AvrII site at position 6719 and a SpeI site at position 8288 facilitated cloning. Envelope fragment B is 1833 bp in length.

### Envelope Fragment C

The following oligonucleotides were used to amplify the 3' distal fragment of the *env* gene.

5'- TTAGTTACATTAGAGCATCAAG-3' [SEQ. I.D. NO. 5] (Coding strand, 8264-V)

5'-TTCTAGATCTTCAGGGTCCCAATACTC-3' [SEQ. I.D. NO. 6] (Complementary
strand, 9145-C)

Primer 8264-V corresponds to nucleotides 8264-8285 of FIV strain 14, and primer 9145-C (underlined region) corresponds to nucleotides 9126-9145 of FIV strain PPR. Primer 9145-C has a synthetic BglII site near the 5' end to facilitate cloning. An SpeI site located at position 8288 also facilitated cloning. Envelope fragment C is 880 bp in length.

In each case, PCR was performed for 35 cycles of 1 min 30 see at 94°C, 2 min at 56°C, and 2 min at 72°C, followed by one cycle of 8 min at 72°C. Each envelope fragment was isolated by gel electrophoresis and cloned into plasmid pSL1190 using standard methods (Maniatis et al., *Molecular Cloning: A Laboratory Manual*, 1982, Cold Spring Harbor Press).

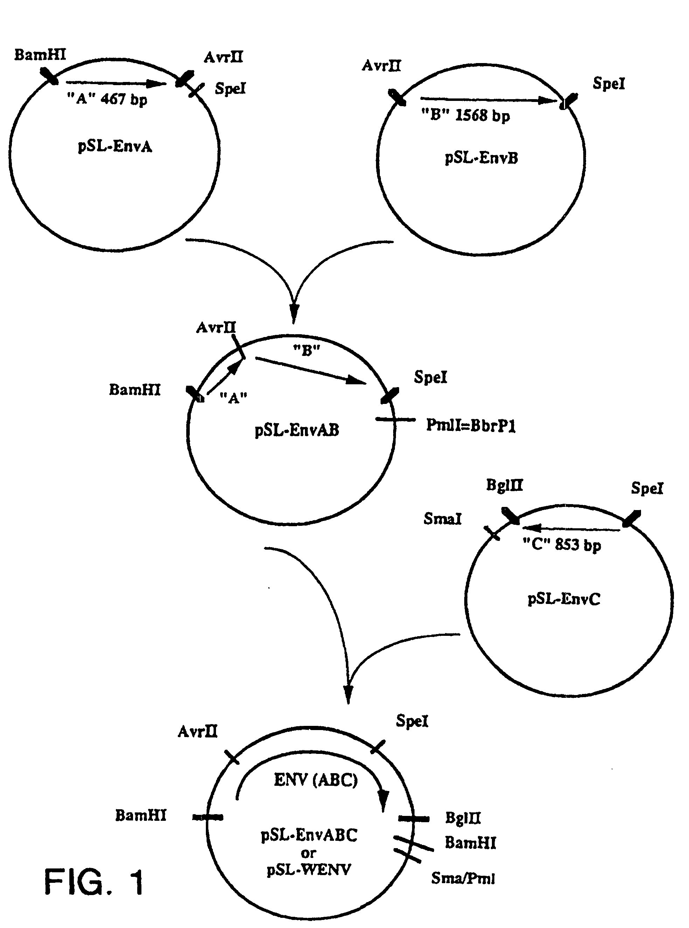

Initially, each fragment was cloned into pSL1190, after which the three fragments were spliced together to re-create a full length envelope gene. For this purpose, the Envelope A plasmid was digested with BamHI and AvrII, the envelope B plasmid was digested was with AvrII and SpeI, and the envelope C plasmid was digested with SpeI and BglII. Subsequently, the 1.5 kbp AvrII/SpeI envelope B fragment was ligated into pSL-EnvA that had been digested with AvrII and SpeI to create pSL-EnvAB (Figure 1). The *env*AB fragment codes for the entire surface membrane protein (SU) and the first 63 amino acids from the ammo-terminus of the transmembrane protein (TM) of FIV-NCSU-1, i.e., amino acids 1 - 735 of *env*. However, *env*AB does not contain the transmembrane domain (TM).

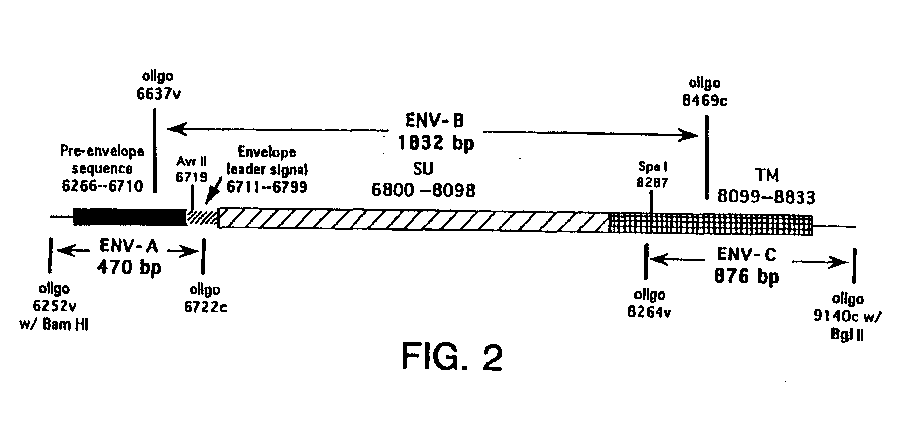

Next, the 0.9 kbp SpeI/SmaI envelope C fragment from pSL-EnvC was ligated into pSL-EnvAB that had been digested with SpeI and BbrPI, to create pSL-EnvABC or pSL-WEnv (Figure 1). The W*Env* fragment codes for the entire *env* open reading frame (SU and TM proteins) of FIV NCSU-1 (Figure 2).

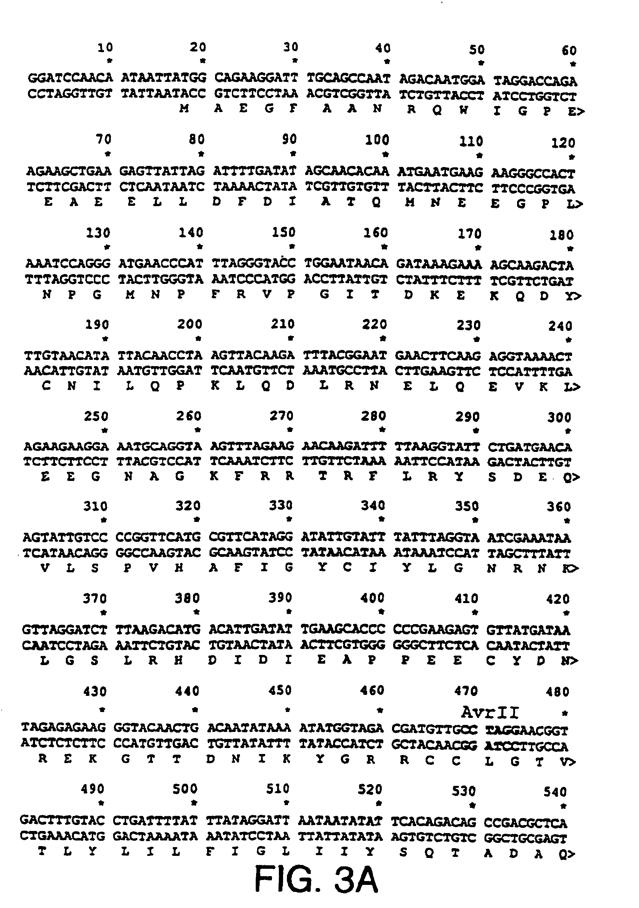

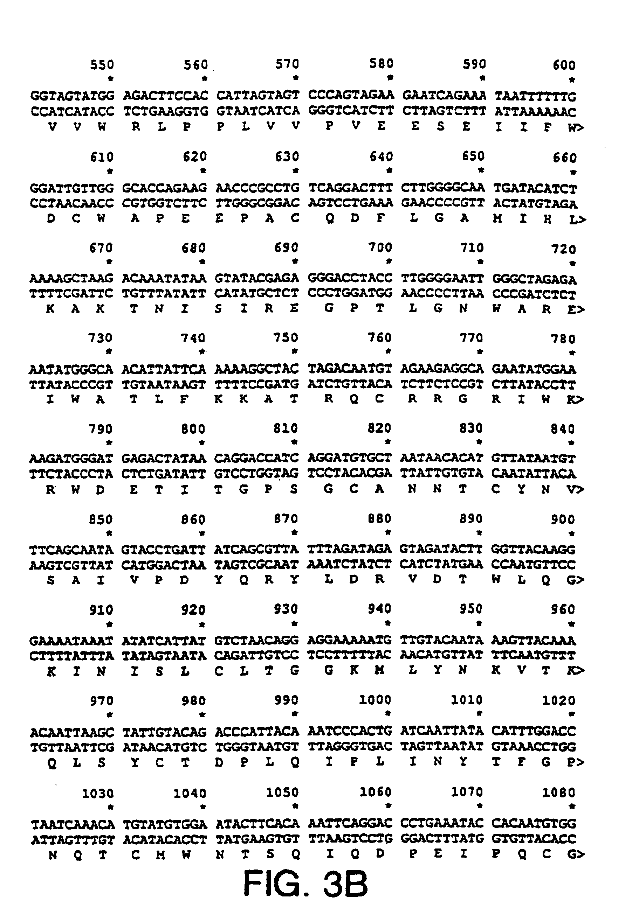

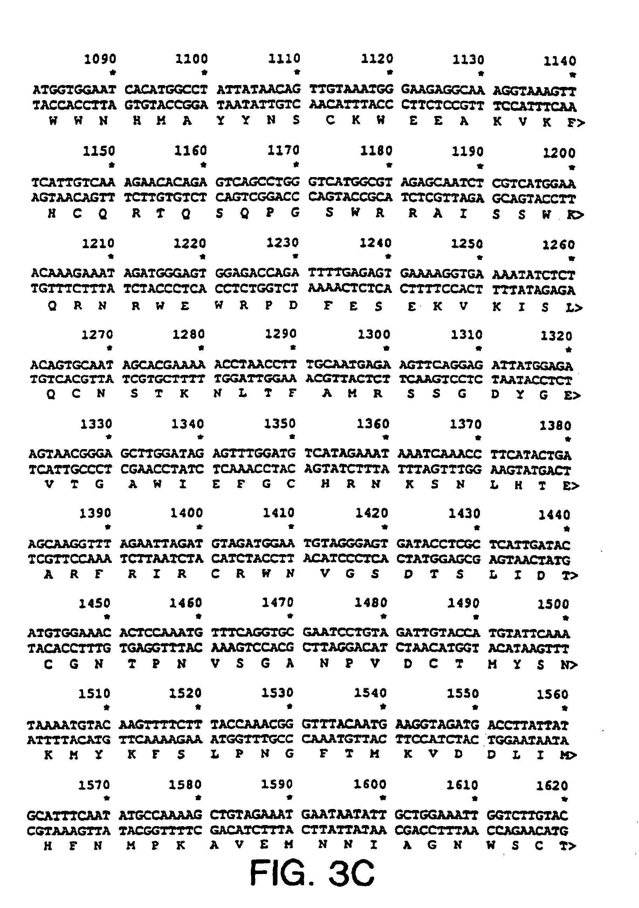

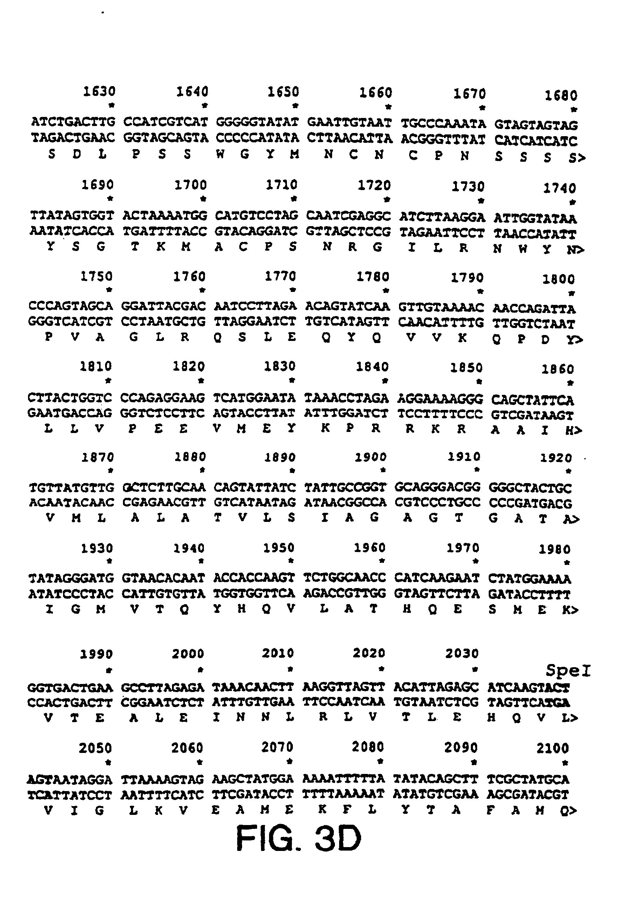

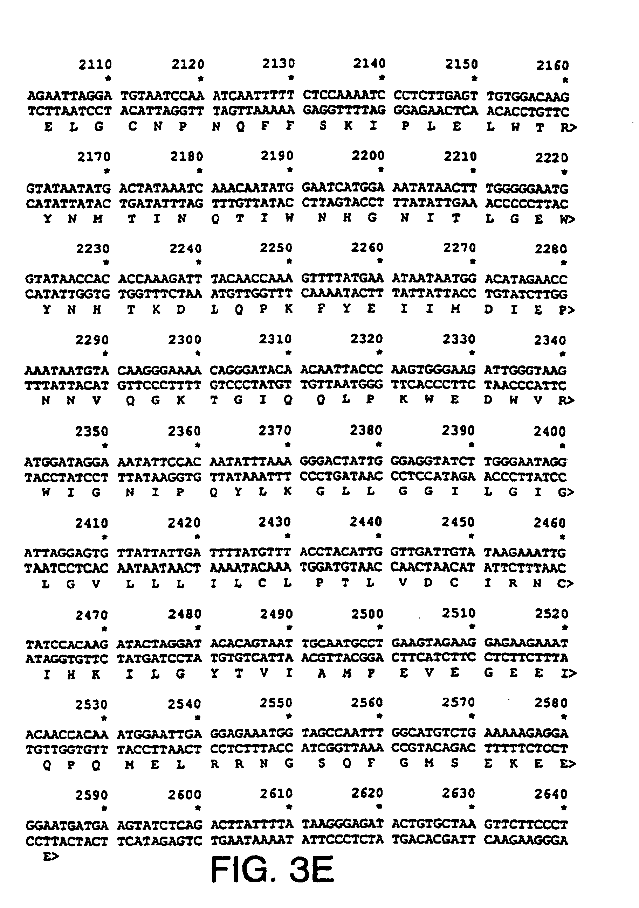

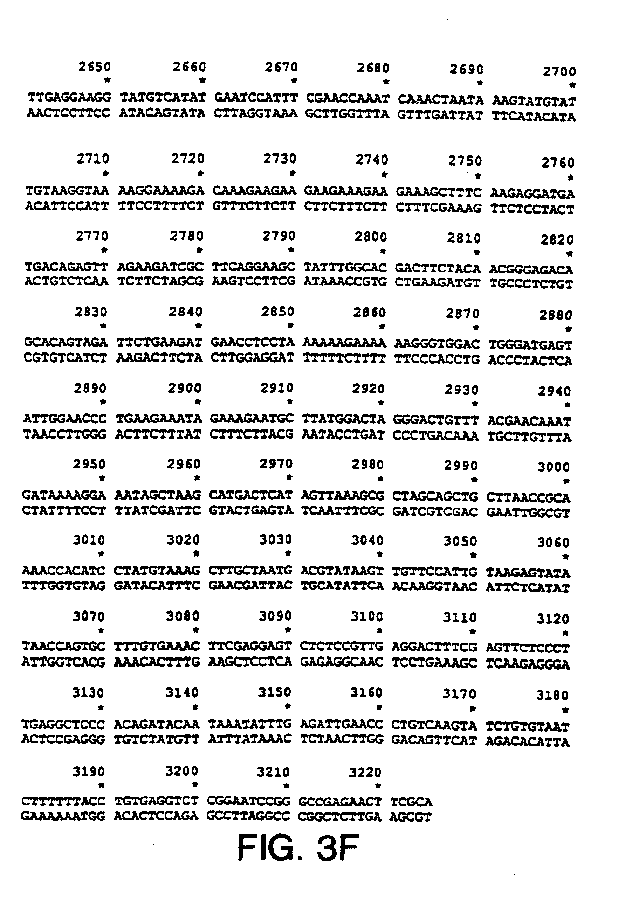

The subcloned genetic elements of FIV-NCSU-1 were sequenced using Sequenase Version 2.0 (United States Biochemical, Cleveland, OH)) as described for double-stranded DNA, and the reactions were analyzed using the ABI automated sequencer (Applied Biosystems, Foster City, CA). Both DNA strands were sequenced to confirm the results. The DNA sequences were analyzed using the MacVector DNA Analysis software (International Biotechnologies, Inc., New Haven, CT). The *env* DNA sequences were analyzed for open reading frames and compared to the previously published DNA sequences of other FIV isolates. The DNA and predicted amino acid sequences of *env* and *env*AB open reading frames of FIV-NCSU-1 are shown in Figure 3.

### C. Cloning of The FIV GAG Gene

The *gag* gene of FIV-NCSU₁ was amplified using PCR and the following oligonucleotide primers:

5'-CAATTCTAGAGAGACTCTACAGCAACATG-3' [SEQ. I.D. NO. 7] (Coding strand,
610-V)

5'-TAATAGATCTGGCCTCTTTTCTAATGATG-3' [SEQ. I.D. NO. 8] (Complementary
strand, 2026-C)

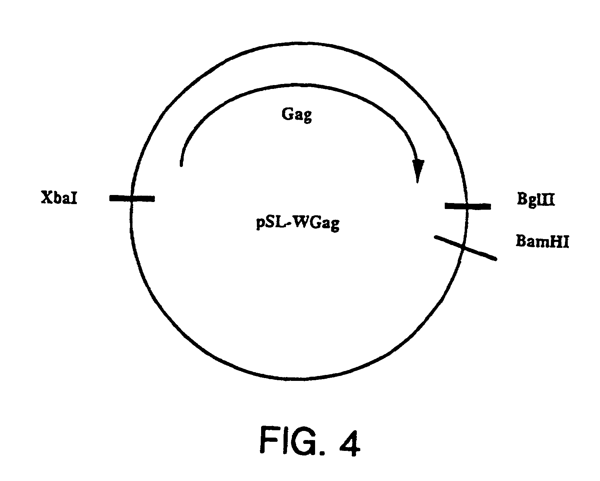

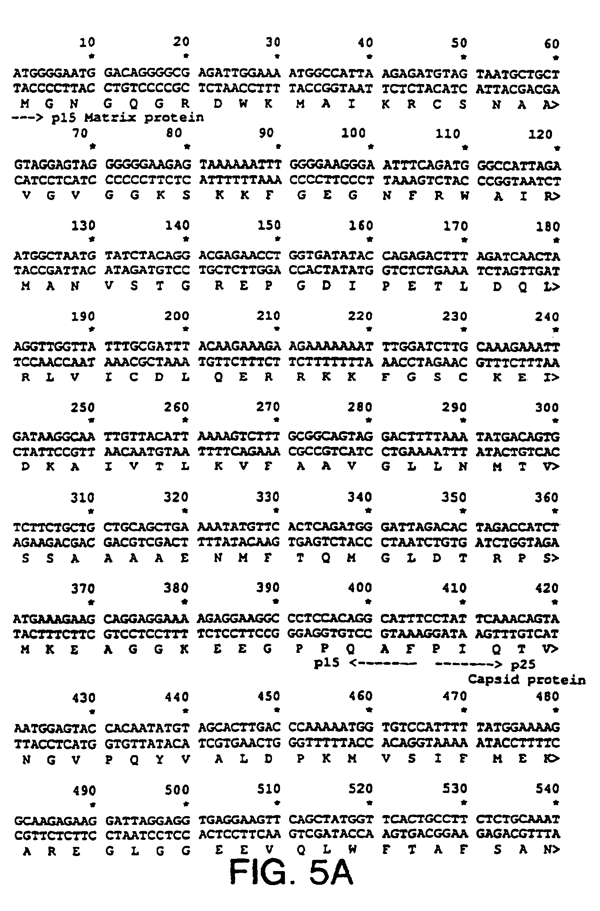

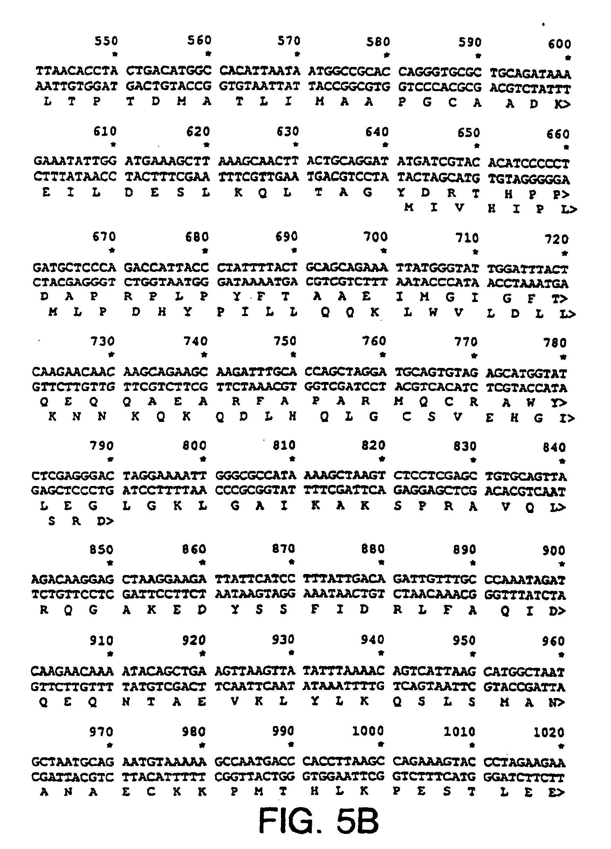

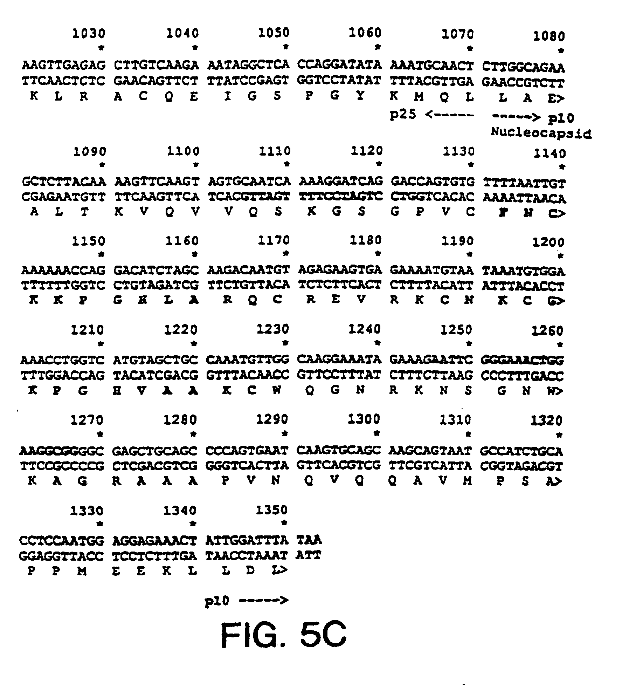

Primers 610-V and 2026-C correspond to nucleotides 610-630 and 2005-2026 of FIV 14 strain, respectively. Primers 610-V and 2026-C have Xbal and BglII restriction enzyme sites, respectively, near their 5' ends to facilitate cloning. The last three nucleotides of primer 610-V correspond to the start codon for *gag* protein translation. PCR was performed for 35 cycles of 1 min 30 sec at 94 C, 2 min at 56 C, and 2 min at 72 C, followed by one cycle of 4 min at 72 C. The 1.4 kbp DNA fragment containing the *gag* gene was purified by gel electrophoresis and cloned into the XbaI/BglII site of pSL1190 to form pSL-WGag (Figure 4). The DNA sequence of FIV-NCSU-1 *gag* is shown in Figure 5.

### Example 2: Preparation of Recombinant Raccoon Poxviruses

### A. Construction of Raccoon Poxvirus Transfer Plasmids

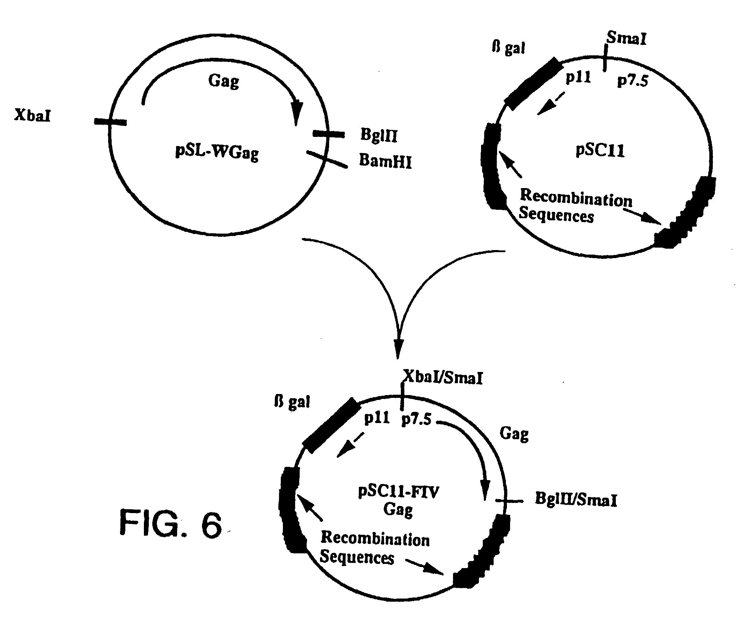

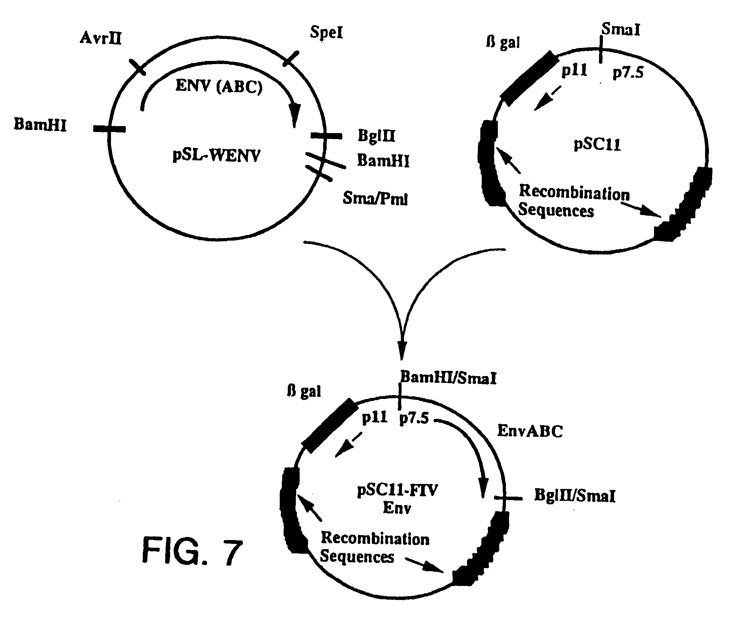

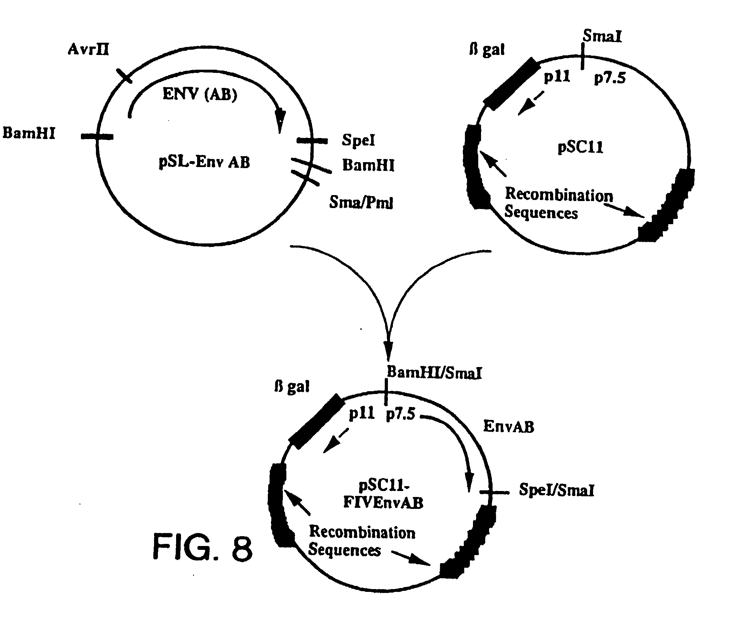

The DNA sequences encoding the *gag, env*, and *env*AB isolated as described in Example 1 were individually subcloned into the poxvirus transfer vector pSC11. The sequence of pSC11 is disclosed in Canadian Patent No. 2132374. which is incorporated by reference. For this purpose, the 1.4 kb XbaI/Bg1II fragment of pSL-WGag (Figure 6), the 2.9 kb BamHI/SpiI fragment of pSL-WEnv (Figure 7) and the 2.1 kb BamHI/SpeI fragment of pSL-EnvAB (Figure 8) were individually isolated and rendered blunt-ended with the Klenow fragment of DNA polymerase, after which each was individually cloned into the SmaI site of pSC11.

### B. Preparation of Recombinant Raccoon Poxviruses

Recombinant raccoon pox viruses (RRPV) bearing the FIV *gag* and *env* genes were prepared as generally described for recombinant vaccinia viruses (Mackett and Smith, *J Gen Virol* **67**:2067-2082, 1986) with some modifications. Monolayers of Vero cells (ATCC CCL 81) that were 80% confluent (approximately 5 x 10⁶ cells in 100 mm tissue culture dishes) were infected with wild-type raccoon pox virus (ATCC VR-838) at a multiplicity of infection (MOI) of 0.1 TCID₅₀/cell in 2 ml of MEM (Eagle's Minimum Essential Medium (Gibco BRL #410-1500) containing 0.05% lactalbumin hydrolysate and 15 mg/ml gentamicin sulfate, adjusted to pH 7.2 with sodium bicarbonate) for 30-60 minutes at 37∞C. The cells were then transfected with either pSC11-FIV *gag*, pSC11-FIV *env*, or pSC11-FIV *env* AB transfer plasmids by cationic liposome-mediated transfection using Transfectam® (Promega Corporation, Madison, Wisconsin) according to manufacturer's instructions. The cells/DNA-liposomes mixture was incubated in 3 ml of MEM containing 5% fetal bovine serum (FBS) overnight at 37∞C (5% CO₂), after which the medium was replaced with 8 ml fresh MEM/5% FBS. The transfected cells were incubated at 37∞C (5% CO₂) until greater than 80% of the cells showed cytopathic effects (approximately 3-4 days). The cells and culture media (viral-cell lysates) were then removed from the plates and subject to two cycles of freeze-thawing before storage at - 70∞C.

### C. Isolation of Recombinant Raccoon Pox Virus Carrying the FIV gag Gene

RRPV carrying the FIV-NCSU₁ *gag* gene (RRPV-FIV *gag*) are isolated and purified from the pSC11-FIV *gag*/Vero cell transfection by standard viral plaque assay methods. Monolayers of Vero cells (50-80% confluent) in 100 mm tissue culture dishes were infected with 2 ml of 10-fold serial dilutions (10⁻¹ to 10⁻³ in MEM) of the viral-cell lysates. After incubation, for 1 hour at 37°C, the media are removed and the infected cells were overlaid with 8-10 ml of 1.25% Noble agar containing MEM/5% FBS. The infected cells were then incubated for 3-4 days at 37°C (5% CO₂), and overlaid again with 4 ml of 1.25% Nobel agar containing 0.5X PBS and 600 ug/ml 5-bromo-4-chloro-3-indolyl-β-D-galactopyranoside (X-gal, United States Biochemical, Cleveland, Ohio). The plates were incubated at 37°C (5% CO₂) for 4-16 hours, until blue viral plaques (β-galactosidase positive) were observed. The recombinant viral plaques were picked with sterile blunt needles attached to a 1 ml (cc) syringe, suspended in 0.5 ml of 0.25 mg/ml trypsin, vortexed vigorously, and incubated at 37°C for 15-30 minutes. The disrupted viral plaques were then inoculated onto 5x10⁵ Vero cells in T-25 cm² flasks and incubated at 37°C (5% CO₂) until greater than 80% CPE was observed. The viral-cell lysates containing RRPV-FIV *gag* were subjected to two cycles of freeze-thawing and stored at -70°C. Five individual RRPV FIV gagclones were selected and plaqued purified four times as described above.

### D. Isolation of Recombinant Raccoon Pox Virus Carrying FIV envAB Gene

RRPV carrying the FIV-NCSU₁ *env*AB gene (RRPV-FIV *env*AB) were isolated and purified from the pSC11-FIV envAB/Vero cell transfection using the methods as described for RRPV-FIV *gag* with some slight modifications. Thymidine kinase deficient (tk-) raccoon pox viruses from the initial viral-cell lysates were selected on tk- Rat-2 cells (ATCC CRL 1764). This was performed by inoculating 1 ml of the initial viral-cell lysate onto a monolayer of Rat-2 cells in a T-75 cm² flask (approximately 5x10⁶ cells) containing 5-bromodeoxyuridine (BrdU) at 30 ug/ml in MEM. The infected monolayer was incubated at 37°C (5% CO₂) for 3-4 days until greater than 70% CPE was observed. The tk- viral-cell lysates were subjected to two cycles of freeze-thawing two times and stored at -70°C. RRPV-FIV *env*AB were isolated and purified from the tk- viral-cell lysates by the standard viral plaque assay as described above for RRPV-FIV *gag* on Vero cells. Five individual RRPV-FIV *env* AS clones were selected and plaque purified five times.

### Example 3: Characteristics of Recombinant FIV-Expressing Raccoon Pox Viruses

### A. Confirmation of FIV gag and evnAB Genes in RRPV by Polymerase Chain Reaction

The presence of the FIV *gag* and *env*AB genes in the RRPVs was confirmed using PCR. 90 µl of a viral-cell lysate was incubated with 10 µl of 10X PCR lysis buffer (1X; 10 mM Tris-HCl buffer, pH 8.5, containing 50 mM KCI, 2.5 mM MgCl₂, 0.5 % Tween 20, 0.3 mg/ml Proteinase K) for 16 hours at 50°C, then boiled for 10 minutes. 10 µl of this lysate was used as a template in the PCR reaction. PCR was performed in 100 µl of 10 mM Tris-HCl buffer, pH 8.3, containing 50 mM KCI, 200 uM of each dNTP, 1.5 mM MgCl₂, 30 pmoles of each primer, and 2.5 Units of AmpliTaq® DNA polymerase (Perkin-Elmer Cetus, Norwalk, CT). The primers used in the PCR for FIV *gag* were: 5'-TATGGAAAAGGCAAGAGAAGGAC-3'[SEQ. I.D. NO. 9] 5'-TCGAGATACCATGCTCTACACTG-3', [SEQ. I.D. NO. 10] corresponding to nucleotides 471-493 and 763-785 of the FIV *gag* open reading frame, respectively. The primers used in the PCR for FIV *env*AB were: 5'-TATGGAAAAGATGGGATGAGACTA-3' [SEQ. I.D. NO. 15] 5'-GTCACTTACCTTCATAGTAAACC-3' [SEQ. I.D. NO. 16] corresponding to nucleotides 857-880 and 1513-1535 of the FTV *env* open reading frame, respectively. The PCR amplifications were performed in a DNA Thermal Cycler (Perkin-Elmer Cetus) by first heating the reaction mixes to 94°C for denaturation, and then 35 cycles of 1 minute at 95°C, 1 minute at 55°C, and 2 minutes at 72°C, and a final incubation of 8 minutes at 72°C. 10 µl of the PCR products were analyzed by electrophoresis in a horizontal-submarine 4 % NuSieve® agarose (FMC BioProducts, Rockland, ME) gel in TAB buffer (40 mM Tris base, 20 mM sodium acetate, 1 mM EDTA, pH 7.2) by applying 5 V/cm for 1-2 hours, and staining with ethidium bromide. PCR amplifications with the FIV *gag* and *env* primers gave expected DNA fragments of 314 and 678 nucleotides, respectively. PCR amplifications using the pSC11 FIV *gag* and *env*AB transfer plasmids served as positive controls. PCR amplifications using wild-type raccoon pox virus-Vero cell lysates served as a negative control.

### B. Confirmation of RRPV FIV gag and envAB Protein Expression by Western Blot Analysis

Confluent monolayers of Vero cells in a T-25 cm² flask (1-2 x 10⁶ cells) were infected with clones of either RRPV-FIV *gag* or RRPV-FIV *env*AB at an M.O.I. of 1 to 10 TCID₅₀ per cell. The infected cells were incubated at 37°C (5% CO₂) for 2-3 days until approximately 80% CPE was observed. 20 µl of the viral-cell lysate was added to 5 µl of 5X Laemmli sample buffer (0.3 M Tris-HCl buffer, pH 6.8, containing 5% SDS, 50% glycerol, 0.4% bromophenol blue, and 3% 2-mercaptoethanol) and heated at 95°C for 5 minutes. The denatured protein samples were separated by SDS/polyacrylamide electrophoresis using a 4-15% gradient polyacrylamide gel (Maniatis et al., Molecular Cloning: A Laboratory Manual, 1982, Cold Spring Harbor Press). After electrophoresis, the proteins were transferred to nitrocellulose filters (Bio-Rad Laboratories, Hercules, CA) by electrotransfer using a Bio-Rad transfer apparatus per manufacturer's instructions. The transfer was performed in 25 mM Tris-HCl buffer, containing 0.2 M glycine and 20% methanol, for 45 minutes at 50V with constant current.

The blot was then screened for FTV *gag* and *env*AB proteins by immunoblot analysis as previously described (Davis et al., *Basic Methods in Molecular Biology,* 1986, Elsevier Science Publishing Company, New York, NY) with some slight modifications. After transfer, the nitrocellulose blot was rinsed in phosphate buffer saline, pH 7.4, containing 0.1 % Tween-20 (PBS-TW), and non-specific sites were blocked by incubating the blot in PBS containing 1 % bovine serum albumin (PBS-BSA) at 4°C overnight, followed by a 15 minute wash in PBS-TW. The blot was then incubated for 30 minutes at room temperature with goat anti-FIV IgG diluted 1:100 in PBS-TW containing 1 % BSA (PBS-TW-BSA), followed by four 5 minute washes in PBS-TW. Next, the blot was incubated for 30 minutes at room temperature with a biotin-labeled mouse-anti-goat IgG antibody (secondary antibody) (Kirkegaard & Perry Laboratories Inc., Gaithersburg, MD) diluted 1:2000 in PBS-TW-BSA, followed by four 5-minute washes in PBS-TW. Antigen-antibody complexes were detected by, incubating the blot for 30 minutes at room temperature with horseradish peroxidase-conjugated streptavidin (Kirkegaard & Perry Laboratories Inc.) diluted 1:1000 in PBS-TW, washing four times for 5 minutes each in PBS-TW, and visualizing with peroxidase chromogenic substrate (Kirkegaard & Perry Laboratories Inc.). Sucrose-gradient purified FIV and wild-type raccoon pox virus/Vero cell lysates were used as the positive and negative controls for the immunoblot analysis, respectively.

Goat anti-FIV antibodies were prepared as follows. FIV NCSU₁ was grown in peripheral blood lymphocytes and concentrated using a hollow fiber apparatus to a concentration of about 10⁶ TCID₅₀/ml. The concentrated virus stock was mixed with an oil adjuvant such as OW3 in a ratio of 1:1 (v:v), and the emulsion was used to inoculate goats six times, at intervals of 3-4 weeks. At monthly intervals, the goats were bled and the serum was tested for the presence of anti-FIV antibodies.

### C. Confirmation of RRPV FIV gag and envAB Protein Expression by Immunofluorescence Assay

Confluent monolayers of Vero cells in 96-well plates (1-2 x 10⁴ cells/well) are infected with clones of either RRPV-FIV *gag* or RRPV-FIV *env*AB at a multiplicity of infection of 0.1 to 1.0 plaque forming units per cell. Cell infected with wild-type RCNV serve as a negative control. The infected cells are incubated at 37°C (5% CO₂) for 1 day until approximately 20% CPE is observed. The cells are then washed three times with PBS and fixed with 80% acetone at 4°C for ten minutes. Next, the cells are rehydrated with PBS and incubated with a monoclonal antibody (IgG) against either FIV *gag* or *env* surface membrane proteins for 30 minutes at room temperature. FIV antigen/FIV antibody complexes are detected using a FITC-conjugared goat anti-mouse IgG (Kirkegaard & Peny Laboratories Inc., Gaithersburg, MD) and fluorescence microscopy.

### D. Raccoon Poxvirus Titration

Virus preparations were pre-treated by dilution into an equal volume of 0.5% trypsin and incubation at 37°C for 30 minutes in order to release virus from inclusions. Serial dilutions (10-fold) of virus were then prepared in MEM and were inoculated (100 µl/well) in replicates of five onto Vero cells (1 x 10⁴ cells in 100 µl per well) in a 96 well plate. Plates were incubated for 3-5 days at 37°C (5% CO₂) and observed for cytopathology typical of raccoon poxvirus. Viral infectivity titers were calculated as 50% endpoints based on cytopathology using the methods of Reed and Muench (Reed and Muench, *The Arnerican Journal of Hygiene* **27**: 493, 1938).

### Example 4: Preparation of Vaccines Based on Recombinant Pox Viruses

### A. Preparation of Master Seeds of RRPV-FIV gag and envAB Viruses

A single clone of each recombinant virus that showed significant recombinant protein expression (by the method described in Example 3 above) was selected for large-scale expansion to serve as a master seed virus. All recombinant virus expansions and titrations were done on Vero cells in MEM containing 2.5% FBS. Each plaque-purified virus clone was expanded by inoculating a confluent monolayer of Vero cells in a T-150 cm² flask (1x10⁷ cells) with 1 ml of viral-cell lysate (approximately 10⁷ infectious virus particles), and incubating at 37°C (5% CO₂) until 100% CPE was observed (2-3 days). This viral-cell lysate served as a pre-master seed virus stock and was used to obtain the master seed virus. The pre-master seed of each recombinant virus was titrated on Vero cells and a TCID₅₀ was determined. The master seed viruses for RRPV-FIV *gag* and RRPV-FIV *env*AB were grown on Vero cells using an MOI of 0.01 and 0.1, respectively. Three roller bottles of confluent Vero cells were infected for each of the master seed viruses using MEM media supplemented with 2.5% fetal bovine serum and incubated for approximately 3 days at 37°C. Infected culture supernatant fluids were harvested, and seed viruses were aliquoted into 1.5 ml ampules, which were sealed and stored in a liquid nitrogen freezer.

### B. Preparation of Vaccines

Vero cells (3 x 10⁷) were seeded into 850 cm² roller bottles in 200 ml of growth media (MEM containing 0.5% lactalbumin hydrolysate and 5% heat-inactivated fetal bovine serum) and incubated for 18 hours at 37°C. The next day, the media were removed from the cells and replaced with 50 ml of RRPV-FIV *gag* at a multiplicity of infection of 0.01 in infection media (MEM containing 0.5 % LAH and 2.5% heat-inactivated fetal bovine serum). The virus used was at the fourth passage beyond the master seed preparation. Virus was allowed to adsorb to the cells for 30 minutes at 37°C, after which the volume of medium was adjusted to 150 ml per roller bottle. Roller bottles were incubated at 37°C until 100% cytopathology was evident (3 days). A virus/cell lysate was then prepared and stored frozen (-70°C). The virus titer of RRPV-FIV *gag* was determined to be 10^{7 4} TCID₅₀/ml.

Recombinant raccoon poxviruses expressing the FTV *env*AB gene fragment were prepared in the same manner, except that a multiplicity of infection of 0.1 was used. The virus was at the fourth passage beyond the master seed preparation. The RRPV-FIV *env*AB preparation was titered and found to contain 10^{6.4} TCID₅₀/ml.

Wild type raccoon poxvirus was grown using the same methods as described above. This virus preparation was found to contain 10^{7.7} TCID₅₀/ml.

### Example 5: Test of Efficacy of FIV Vaccines Based on Recombinant Pox Viruses

### A. Vaccination

Thirty 6-7 month old cats (specific pathogen-free, Harlan Sprague Dawley, Madison, WI), fifteen males and fifteen females, were used. Cats were divided into six groups and vaccinated twice, 21 days apart, as indicated below:

**Table 1.**

| **Assignment of Groups for Vaccination** | | | | | |
|---|---|---|---|---|---|
| Group | # Cats | Vaccine | Volume | Virus Dose(TCID₅₀) | Route* |
| 1 | 5 | RRPV-FIV gag | 1 mL | 10^{7.4} | SC |
| 2 | 5 | RRPV-FIV gag | 1 mL | 10^{7.4} | IM |
| 3 | 5 | RRPV-FIV *env*AB | 3 mL | 10^{6.9} | SC |
| 4 | 5 | RRPV-FIV *env*AB | 2 mL | 10^{6.7} | IM |
| 5 | 5 | RRPV-FIV *gag*(1 ml)+ | 4 mL | 10^{7.4} (*gag*) | SC |
| | | RRPV-FIV *envAB* | 3 mL | 10^{6.9} (*envAB*) | SC |
| 6 | 5 | Wild Type raccoon pox virus | 1 mL | 10^{7.7} | SC |

| | | | | | |
|---|---|---|---|---|---|
| * SC = Subcutaneous Vaccination IM = Intramuscular Vaccination | | | | | |

### B. Experimental Design

Twenty-five cats were vaccinated with the recombinant raccoon poxvirus vaccines as indicated in Table 1. Five cats were administered a similar titer of wild type raccoon poxvirus to serve a negative controls. Two vaccinations were administered 21 days apart. Subcutaneous vaccinations were administered in the nape of the neck, and intramuscular vaccinations were administered in a rear thigh. Four weeks following the second vaccination, all cats were challenged with the NCSU-1 strain of FIV and monitored for viremia and evidence of lymphocyte population changes as described below. Eleven months following FIV challenge, cats in Groups 1, 2, 3, 4, and 6 were challenged with *Toxoplasma gondii* and monitored for 48 days for clinical signs of disease.

### C. FIV Challenge

Four weeks following the second vaccination, all of the cats were challenged subcutaneously with 10 cat ID₅₀ units of the NCSU₁ isolate of FIV(1:1000 dilution of lot # 021891). Whole blood was obtained from the cats prior to challenge, and periodically after challenge, in order to assess virus infection parameters as follows:

### 1. Detection of Viremia

Culture isolation of FIV was performed as described previously (Wasmoen et al., *Vet. Irrrmuno. Immunopath*. **35**:83 1992). Monoauclear cells were isolated from whole blood using Percoll™ (Pharmacia Biotech, Piscataway NJ) gradients. 5 x 10⁵ cells from FIV-challenged cats were cultured with 1 x 10⁶ mononuclear cells isolated from uninfected cats. Cultures were fed with RPMI media every 7 days and supernatants tested for the presence of FIV by an enzyme-linked immunosorbent assay (ELISA) that detects FIV p25 antigen (Petcheck ELISA, IDEXX, Portland ME).

### 2. Lymphocyte Subsets

Leukocytes were isolated from whole blood using Histopaque™ (Sigma Chemical Company, St. Louis MO) and lymphocyte subsets quantitated by staining the cells with antibodies specific to CD4 (monoclonal antibody CAT30A), CD8 (monoclonal antibody FLSM 3.357), pan T lymphocytes (monoclonal antibody FLSM 1.572) or B lymphocytes (anti-cat IgG) followed by FACS analysis. These monoclonal antibodies are described elsewhere (Tompkins et al. *Vet. Immunol. Immunopathol*. **26**:305, 1990) and the flow cytometry procedure is the same as previously described (R. V. English et al. *J. Infect. Dis*. 170:543, 1994). CD4:CD8 ratios were calculated.

### D. Toxoplasma gondii Challenge

Tacheozoites of the ME49 strain of *T. gondii* that were frozen in 10% glycerol were inoculated intraperitoneally into Swiss mice (Charles Rivers Laboratories) and serially passed in mice according to published procedures (Davidson et al., *Am. J. Pathol*. **143**:1486, 1993). Tacheozoites harvested from peritoneal fluids of mice were enumerated using a hemacytometer. Cats were tranquilized using ketamine hydrochloride and inoculated with 50,000 fresh tachyzoites into the right common carotid artery that had been surgically isolated. Cats were monitored for clinical signs of disease, including ocular discharge, nasal discharge, dyspnea, fever, depression, and weight loss for 3 days prior to and 48 days following *T. gondii* inoculation.

Clinical signs follow *T. gondii* challenge were scored as follows:

| Clinical Sign | | Score |
|---|---|---|
| Fever | 103.0 to | 1 point per day |
| | 103.9°F | |
| | 104.0 to | 2 points per day |
| | 104.9°F | |
| | ≥105.0°F | 3 points per day |
| *(Temperatures were not scored until ≥1°F above baseline.)* | | |
| Depression/Lethargy | | 1 point per day |
| Dehydration | | 2 points per day |
| Nasal Discharge | | 1 point per day |
| Ocular Discharge | | 1 point per day |
| Respiratory Distress: | | |
| Tachypnea | | 2 points per day |
| Dyspnea | | 4 points per day |

### E. RESULTS

At one month following inoculation with the NCSU-1 strain of FIV, 60% of the control cats were found to be viremic (Table 2). Cats vaccinated with RRPV-FIV *gag* were all negative for FIV, 40% of the cats vaccinated with RRPV-FIV *env*AB were virus positive, and 20% of the cats vaccinated with a combination of these two viruses were viremic (Table 2). Therefore, the ability of the test vaccines to prevent viremia at this time point varied from 33% to 100% (Table 3).

At three months after FIV challenge, 80% of the control cats were found to be virus positive (Table 2). Similarly, FIV could be isolated from peripheral blood mononuclear cells of nearly all vaccinated cats using this very sensitive method (Table 2).

With respect to immune status, 80% of the control cats showed evidence of CD4:CD8 lymphocyte ratio inversions (i.e. ratios less than 1.0) at three months (Table 2). In contrast, only 30% of the RRPV-*gag* vaccinated cats had evidence of significant CD4:CD8 inversions, and the RRPV-FIV *env*AB vaccinates were similarly protected from this lymphocyte subset change (Table 2). Cats vaccinated with a combination of the two recombinant viruses were not significantly different from the controls (i.e. 80% showed CD4:CD8 inversions) at 3 months after challenge (Table 2).

At 9 months after FIV challenge, 100% of the control cats were FIV infected, and all showed CD4:CD8 inversions (Table 2). A large percentage of the vaccinated cats were also shown to be viremic at this time point. However, only 50% of the RRPV-FIV *gag* vaccinates and 20% of the RRPV-FIV *env*AB vaccinates showed evidence of CD4:CD8 inversions at this time point. *Therefore, these two vaccines showed a significant ability to prevent the CD4:CD8 lymphocyte ratio changes associated with FIV infection even though the cats appeared to be viremic (Table 3).*

In order to determine whether CD4:CD8 lymphocyte subset inversions signified a deterioration in the immune system of cats following FIV infection (and, conversely, that lack of inversion in vaccinates signified maintenance of immune function), vaccinated and control cats (from groups 1, 2, 3, 4, and 6) were challenged with *Toxoplasma gondii.* This parasite causes subclinical infections in normal cats, but has been reported to cause severe disease in cats that are immunocompromised due to FIV infection (Davidson et al., *Am. J. Pathol*. **143**:1486, 1993). Following *T. gondii* challenge, control cats displayed ocular discharge, nasal discharge, dyspnea, and fever. The average total clinical score for control cats was 15.6 (Table 4). By comparison, there was a 41 % reduction in clinical disease scores in RRPV-FIV gag vaccinated cats, related to reductions in clinical signs of ocular discharge and dyspnea (Table 4). The clinical picture following *T. gondii* challenge was even less severe in RRPV-FIV *env*AB vaccinated cats. This group showed a 92% decrease in ocular signs, 75% decrease in nasal discharge, 73% reduction in dyspnea, and 58% decrease in overall clinical scores (Table 4). Further, 80% of the control cats displayed weight loss in the first 14 days after challenge, compared to weight loss in only 44% of the RRPV-FIV *gag* vaccinates and 50% of the V-FIV *env*AB vaccinates. Therefore, control cats were more susceptible to induction of disease by this opportunistic pathogen than vaccinated cats.

These data suggest that vaccination altered the progression of clinical disease caused by this virus (i.e. induction of immune suppression). This is indicated by a lower rate of CD4:CD8 inversions in vaccinated cats and by a decreased susceptibility to infection with the opportunistic pathogen *T. gondii*.

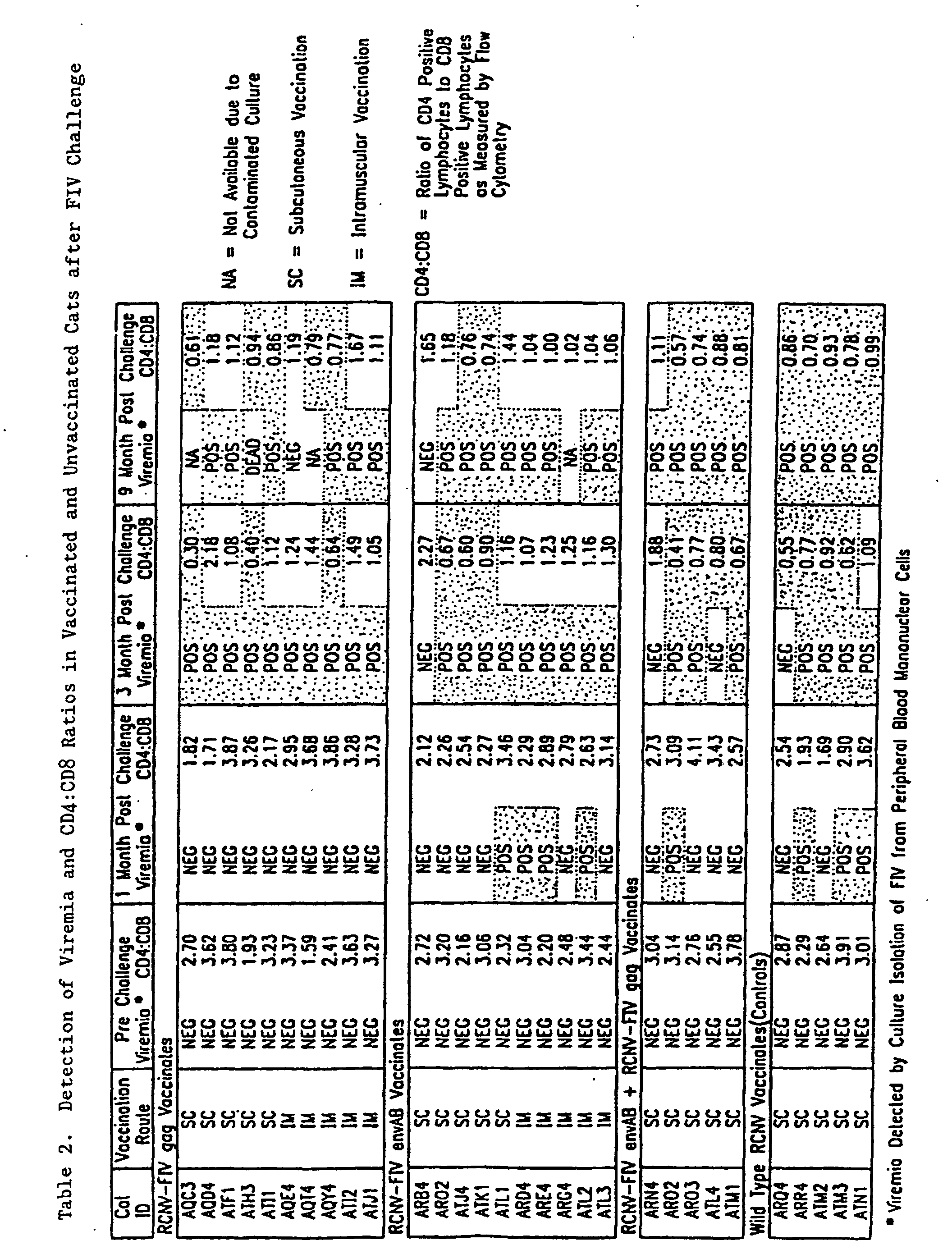

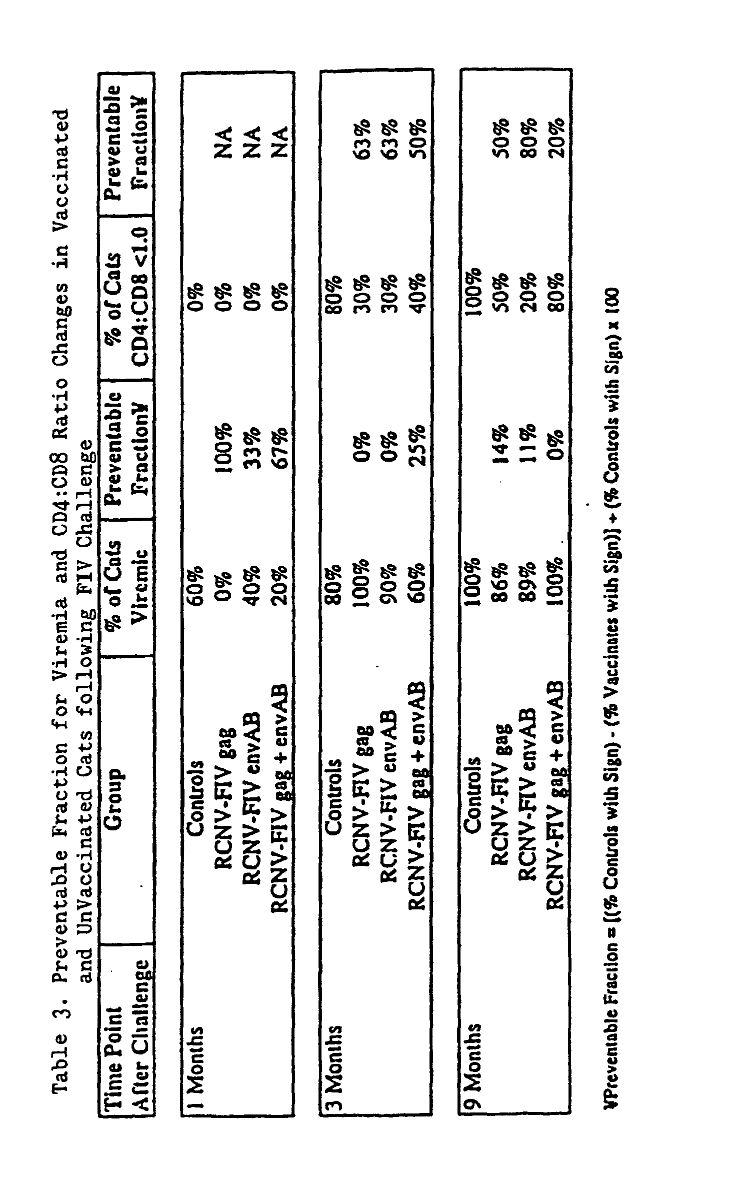

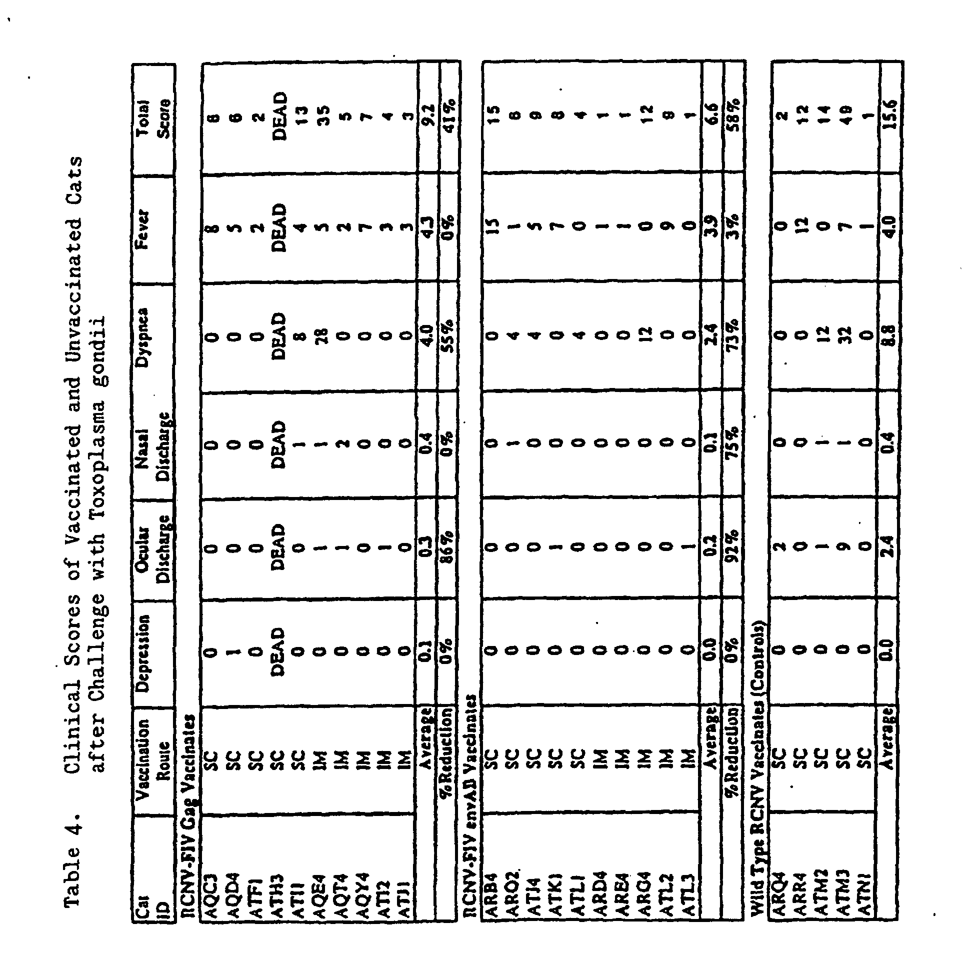

## Claims

1. A recombinant raccoon poxvirus having at least one internal gene comprising a DNA sequence encoding a polypeptide consisting of amino acids 1-735 of the envelope protein of a Feline immunodeficiency Virus (FIV).

2. The recombinant raccoon poxvirus of claim 1 wherein said internal gene encodes the FIV envelope protein having the amino acid sequence as set out in SEQ. ID No. 12.

3. The recombinant raccoon poxvirus of claim 1 wherein said internal gene encodes amino acids 1-735 of the FIV envelope protein having the amino acid sequence as set out in SEQ. ID No. 12.

4. A vaccine comprising the recombinant raccoon poxvirus according to claim 1 and a pharmaceutically acceptable carrier or diluent.

5. The vaccine of claim 4 further comprising a pharmaceutically acceptable adjuvant.

6. The vaccine of claim 4 wherein said internal gene encodes the FIV envelope protein having the amino acid sequence as set out in SEQ. ID No. 12.

7. The vaccine of claim 4 wherein said internal gene encodes amino acids 1-735 of the FIV envelope protein having the amino acid sequence as set out in SEQ. ID No. 12.

8. The vaccine of claim 4 further comprising immunogens derived from viruses selected from the group consisting of feline leukemia virus, feline panleucopenia virus, feline rhinotracheitis virus, feline calicivirus, feline infectious peritonitis virus, feline herpesvirus, feline enteric coronavirus, or mixtures thereof.

9. The vaccine of claim 4 further comprising inactivated or attenuated feline *Chlamydia psittaci, Microsporum canis*, or mixtures thereof.

10. A vaccine comprising:
the recombinant raccoon poxvirus according to claim 1;
a second recombinant raccoon poxvirus having at least one internal gene comprising a DNA sequence encoding a *gag* protein of Feline Immunodeficiency Virus (FIV); and
a pharmaceutically acceptable carrier or diluent

11. The vaccine of claim 10 further comprising a pharmaceutically acceptable adjuvant.

12. An isolated DNA molecule encoding amino acids 1-735 of the Feline Immunodeficiency Virus (FIV) envelope protein according to SEQ ID No. 12.

13. An isolated DNA molecule encoding amino acids 1-735 of the Feline Immunodeficiency Virus (FIV) envelope protein according to SEQ ID No. 12, for use in medicine.

14. Use of the recombinant raccoon poxvirus according to claim 1, in the preparation of a vaccine for the prevention or lessening of disease caused by Feline Immunodeficiency Virus (FIV).

15. The use of claim 14, wherein said internal gene encodes the FIV envelope protein having the amino acid sequence as set out in SEQ. ID No. 12.

16. The use of claim 14, wherein said internal gene encodes amino acids 1-735 of the FIV envelope protein according to SEQ ID No. 12.

## Patentansprüche

1. Rekombinantes Waschbär-Pockenvirus (raccoon poxvirus) mit mindestens einem internen Gen, das eine DNA-Sequenz aufweist, die für ein Polypeptid bestehend aus den Aminosäuren 1-735 des Hüllenproteins eines felinen Immunschwäche-Virus (Feline immunodeficiency Virus, FIV) codiert.

2. Rekombinantes Waschbär-Pockenvirus nach Anspruch 1, wobei das interne Gen für das FIV-Hüllenprotein mit der Aminosäuresequenz gemäß SEQ. ID Nr. 12 codiert.

3. Rekombinantes Waschbär-Pockenvirus nach Anspruch 1, wobei das interne Gen für die Aminosäuren 1-735 des FIV-Hüllenproteins mit der Aminosäuresequenz gemäß SEQ. ID Nr. 12 codiert.

4. Vakzin, umfassend das rekombinante Waschbär-Pockenvirus gemäß Anspruch 1 und ein pharmazeutisch akzeptables Träger- oder Verdünnungsmittel.

5. Vakzin nach Anspruch 4, welches weiters ein pharmazeutisch akzeptables Adjuvans aufweist.

6. Vakzin nach Anspruch 4, wobei das interne Gen für das FIV-Hüllenprotein mit der Aminosäuresequenz gemäß SEQ. ID Nr. 12 codiert.

7. Vakzin nach Anspruch 4, wobei das interne Gen für die Aminosäuren 1-735 des FIV-Hüllenproteins mit der Aminosäuresequenz gemäß SEQ. ID Nr. 12 codiert.

8. Vakzin nach Anspruch 4, welches weiters Immunogene aufweist, die von Viren ausgewählt aus der Gruppe bestehend aus felinem Leukämie-Virus, felinem Panleukopänie-Virus, felinem Rhinotracheitis-Virus, felinem Calicivirus, infektiösem felinem Peritonitis-Virus, felinem Herpesvirus, felinem enteralem Coronavirus oder Mischungen davon, stammen.

9. Vakzin nach Anspruch 4, welches weiters inaktivierte oder attenuierte feline Chlamydia psittaci, Microsporum canis oder Mischungen davon aufweist.

10. Vakzin, welches umfasst:
das rekombinante Waschbär-Pockenvirus nach Anspruch 1;
ein zweites rekombinantes Waschbär-Pockenvirus mit mindestens einem internen Gen, das eine DNA-Sequenz aufweist, die für ein gag-Protein des felinen Immunschwäche-Virus (FIV) codiert; und
ein pharmazeutisch akzeptables Träger- oder Verdünnungsmittel.

11. Vakzin nach Anspruch 10, welches weiters ein pharmazeutisch akzeptables Adjuvans aufweist.

12. Isoliertes DNA-Molekül, das für die Aminosäuren 1-735 des felinen Immunschwäche-Virus(FIV)-Hüllenproteins gemäß SEQ. ID Nr. 12 codiert.

13. Isoliertes DNA-Molekül, das für die Aminosäuren 1-735 des felinen Immunschwäche-Virus(FIV)-Hüllenproteins gemäß SEQ. ID Nr. 12 codiert, zur Verwendung in der Medizin.

14. Verwendung des rekombinanten Waschbären-Pockenvirus nach Anspruch 1 bei der Herstellung eines Vakzins zur Verhinderung oder Abschwächung einer durch das feline Immunschwäche-Virus (FIV) verursachten Erkrankung.

15. Verwendung nach Anspruch 14, wobei das interne Gen für das FIV-Hüllenprotein mit der Aminosäuresequenz gemäß SEQ. ID Nr. 12 codiert.

16. Verwendung nach Anspruch 14, wobei das interne Gen für die Aminosäuren 1-735 des FIV-Hüllenproteins gemäß SEQ. ID Nr. 12 codiert.

## Revendications

1. Poxvirus de raton laveur recombiné ayant au moins un gène interne comprenant une séquence d'ADN codant un polypeptide constitué des acides aminés 1-735 de la protéine d'enveloppe d'un virus de l'immunodéficience du chat (FIV).

2. Poxvirus de raton laveur recombiné selon la revendication 1 dans lequel ledit gène interne code la protéine d'enveloppe de FIV ayant la séquence d'acides aminés comme établie dans SEQ ID NO:12.

3. Poxvirus de raton laveur recombiné selon la revendication 1 dans lequel ledit gène interne code les acides aminés 1-735 de la protéine d'enveloppe de FIV ayant la séquence d'acides aminés comme établie dans SEQ ID NO:12.

4. Vaccin comprenant le poxvirus de raton laveur recombiné selon la revendication 1 et un excipient ou diluant pharmaceutiquement acceptable.

5. Vaccin selon la revendication 4 comprenant en plus un adjuvant pharmaceutiquement acceptable.

6. Vaccin selon la revendication 4 dans lequel ledit gène interne code la protéine d'enveloppe de FIV ayant la séquence d'acides aminés comme établie dans SEQ ID NO:12.

7. Vaccin selon la revendication 4 dans lequel ledit gène interne code les acides aminés 1-735 de la protéine d'enveloppe de FIV ayant la séquence d'acides aminés comme établie dans SEQ ID NO:12.

8. Vaccin selon la revendication 4 comprenant en plus des immunogènes dérivés de virus choisis parmi le groupe constitué du virus de la leucémie du chat, du virus de la panleucopénie infectieuse du chat, du virus de la rhinotrachéite du chat, du calicivirus du chat, du virus de la péritonite infectieuse du chat, de l'herpèsvirus du chat, du coronavirus entérique du chat ou des mélanges de ceux-ci.

9. Vaccin selon la revendication 4 comprenant en plus Chlamydia psittaci, Microsporum canis, du chat, inactivés ou atténués, ou des mélanges de ceux-ci.

10. Vaccin comprenant :
le poxvirus de raton laveur recombiné selon la revendication 1 ;
un second poxvirus de raton laveur recombiné ayant au moins un gène interne comprenant une séquence d'ADN codant une protéine gag du virus de l'immunodéficience du chat (FIV) ; et
un excipient ou diluant pharmaceutiquement acceptable.

11. Vaccin selon la revendication 10 comprenant en plus un adjuvant pharmaceutiquement acceptable.

12. Molécule d'ADN isolée codant les acides aminés 1-735 de la protéine d'enveloppe du virus de l'immunodéficience du chat (FIV) selon SEQ ID NO:12.

13. Molécule d'ADN isolée codant les acides aminés 1-735 de la protéine d'enveloppe du virus de l'immunodéficience du chat (FIV) selon SEQ ID NO:12 pour l'utilisation en médecine.

14. Utilisation du poxvirus de raton laveur recombiné selon la revendication 1 dans la préparation d'un vaccin pour la prévention ou l'atténuation de maladie provoquée par le virus de l'immunodéficience du chat (FIV).

15. Utilisation selon la revendication 14, dans laquelle ledit gène interne code la protéine d'enveloppe de FIV ayant la séquence d'acides aminés comme établie dans SEQ ID NO:12.

16. Utilisation selon la revendication 14 dans laquelle ledit gène interne code les acides aminés 1-735 de la protéine d'enveloppe de FIV selon la SEQ ID NO:12.
